# EUROPÄISCHE PATENTANMELDUNG

(11) **EP 1 917 937 A1**
(43) Veröffentlichungstag der Anmeldung: **07.05.2008**
(21) Anmeldenummer: 06022937.4
(22) Anmeldetag: 03.11.2006
(51) Int. Cl.: A61F 9/02

(54) **Sportbrille, insbesondere Skibrille**

(71) Anmelder: Egger, Christoph, 6290 Mayrhofen (AT)
(72) Erfinder: Egger, Christoph, 6290 Mayrhofen (AT)
(74) Vertreter: Tiesmeyer, Johannes

(57) **Zusammenfassung**

Die Erfindung stellt eine Sportbrille bereit, umfassend einen Durchsichtbereich (12) und einen den Durchsichtbereich (12) zumindest teilweise umlaufenden Rahmen (10), wobei an dem Rahmen (10) ein wulstartiges Zierelement (42) befestigt ist und der Rahmen (10) zumindest an seiner Oberseite mindestens einen Halteabschnitt aufweist, welcher einen oberen Zierelementabschnitt (52) des Zierelements (42) derart abstützt, dass der größte Teil der vorn liegenden Seite des oberen Zierelementabschnitts (52) bei Betrachtung in einer zur Durchsichtrichtung der Sportbrille entgegengesetzten Richtung sichtbar freiliegt.

## Beschreibung

Die vorliegende Erfindung betrifft eine Sportbrille umfassend einen Durchsichtbereich und einen den Durchsichtbereich zumindest teilweise umlaufenden Rahmen.

Sportbrillen dieser Art finden besonders im Freizeitsportbereich, aber auch im Wettkampfsport Anwendung und erfüllen dort zumeist funktionelle Aufgaben zum Schutz der Augen gegenüber Sonnenlichteinstrahlung oder Witterungseinflüssen. Zu solchen Sportbrillen zählen beispielsweise Ski-oder Snowboardbrillen, bei welchen der Durchsichtbereich aus einem durchgehenden Sichtfenster für beide Augen gebildet ist und welche zumeist mittels eines elastischen Haltebands am Kopf des Trägers gehalten werden (sog. "goggles"). Üblich sind jedoch auch Sportbrillen, bei welchen der Durchsichtbereich zwei getrennte Sichtfenster für beide Augen aufweist, oder bei welchen Kopfhalterungsmittel in der Art von Brillenbügeln vorgesehen sind.

Die Konstruktion und Vermarktung von Sportbrillen der o.g. Art richtet sich zum einen nach funktionellen Gesichtspunkten, wie etwa einem möglichst guten Schutz gegen Sonnenlichteinstrahlung (UV-Strahlung), Wind, Schnee und Regen, einem hohen Tragekomfort und guter Stabilität. Andererseits liegt jedoch ständig wachsendes Augenmerk auf einer Verbesserung der gestalterischen Möglichkeiten zur Schaffung eines besonderen äußeren Erscheinungsbilds der Sportbrille. So ist es insbesondere im Freizeitbereich erwünscht, der Sportbrille ein besonders markantes bzw. ästhetisches Erscheinungsbild zu verleihen und Markensymbole bzw. -bezeichnungen an der Sportbrille darzustellen. Für Gestaltungen dieser Art werden herkömmlich das Kopfhalteband oder ggf. die Brillenbügel mit entsprechenden Mustern oder Schriftzügen versehen, oder es kommen verschiedene Farben für die Verspiegelung des Durchsichtbereichs oder den den Durchsichtbereich umgebenden Rahmen zum Einsatz.

Veränderungen des Designs der Sportbrille unter Verwendung dieser Elemente unterliegen jedoch den durch die Funktion und Materialien dieser Elemente auferlegten Beschränkungen. So sollte beispielsweise der Rahmen der Sportbrille aus einem leichten und in gewissem Maße flexiblen Material gebildet sein (beispielsweise TPU), während das Kopfhalteband aus einem elastischen Textilband gebildet sein sollte. Gleichzeitig ist es wünschenswert, dass die Sportbrille insgesamt möglichst stabil und widerstandsfähig gegenüber mechanischen Beanspruchungen ist, um auch unter den besonderen Bedingungen während oder im Umfeld des sportlichen Einsatzes eine möglichst lange Lebensdauer der Brille zu erreichen.

Aufgabe der vorliegenden Erfindung ist es, eine Sportbrille der o.g. Art bereitzustellen, deren Konstruktion flexiblere Gestaltungsmöglichkeiten des äußeren Erscheinungsbilds ermöglichen, ohne die funktionellen Eigenschaften oder die Stabilität der Sportbrille zu beeinträchtigen.

Gemäß der vorliegenden Erfindung wird diese Aufgabe gelöst durch eine Sportbrille umfassend einen Durchsichtbereich und einen den Durchsichtbereich zumindest teilweise umlaufenden Rahmen, wobei an dem Rahmen ein wulstartiges Zierelement befestigt ist und der Rahmen zumindest an seiner Oberseite mindestens einen Halteabschnitt aufweist, welcher einen oberen Zierelementabschnitt des Zierelements derart abstützt, dass der größte Teil der vorn liegenden Seite des oberen Zierelementabschnitts bei Betrachtung in einer zur Durchsichtrichtung der Sportbrille entgegengesetzten Richtung sichtbar freiliegt.

In dieser Beschreibung sowie den Ansprüchen beziehen sich Positionsangaben wie oben, unten, vorn, hinten, seitlich, vertikal, horizontal, etc. auf die Sichtweise eines Trägers der Sportbrille, der die Sportbrille am Kopf trägt und im Wesentlichen aufrecht steht. Dementsprechend verläuft die Durchsichtrichtung durch die Sportbrille im Wesentlichen horizontal von hinten nach vorn. Eine vorn liegende Seite des oberen Zierelementabschnitts bezeichnet eine vom Brillenträger abgewandte und von außen für eine andere Person sichtbare Seite, während eine hintere Seite oder Rückseite der Sportbrille eine dem Kopf des Trägers zugewandte und von der anderen Person abgewandte Seite bezeichnet.

Die erfindungsgemäße Sportbrille weist ein wulstartiges Zierelement auf, welches zumindest oberhalb des Durchsichtbereichs entlang dem Rahmen verläuft. Das Zierelement ist dabei am Rahmen so gehalten, dass es von vorn gut sichtbar ist und somit in einem Benutzungszustand der Sportbrille, in welchem die Sportbrille am Kopf eines Trägers gehalten ist, deutlich zum äußeren Erscheinungsbild der Sportbrille beiträgt. Es ist dementsprechend möglich, das Zierelement auf verschiedene Art zu gestalten und somit ein gewünschtes markantes Design der Sportbrille bereitzustellen.

Das wulstartige Zierelement dient erfindungsgemäß der Schaffung einer Gestaltungsfläche zur Gestaltung, d.h. Verzierung, der Sportbrille. Es kann prinzipiell darüber hinaus auch funktionelle Aufgaben der Brille übernehmen, beispielsweise einen zusätzlichen Schutz vor Witterungseinflüssen bieten, oder ein Anlagepolster der Sportbrille bereitstellen. Besonders bevorzugt ist jedoch die separate Bereitstellung des Zierelements zusätzlich zu den zur Sicherstellung der Funktionalität der Sportbrille unbedingt notwendigen Elementen, das heißt als reines Zierelement. Durch die Ausbildung des Zierelements in wulstartiger Form und die Anordnung des Zierelements entlang zumindest eines Teils der Oberseite des Rahmens kann das Zierelement optisch auffällig an der Sportbrille angebracht sein, ohne andererseits die Funktionen der Sportbrille zum Schutz der Augenbereiche oder den Tragekomfort negativ zu beeinflussen.

In einer bevorzugten Ausführungsform der Erfindung ist vorgesehen, dass an dem Rahmen ferner ein von dem Zierelement separates Anlagemittel vorgesehen ist, welches dafür eingerichtet ist, in einem Benutzungszustand der Sportbrille zumindest an der Stirn einer die Sportbrille tragenden Person anzuliegen.

In einer solchen Ausführungsform sind das Zierelement und ein Anlagemittel also als separate Elemente bereitgestellt, so dass das hinter dem Rahmen bzw. dem Zierelement von vorn nicht sichtbare Anlagemittel aus einem seiner Funktion angepassten Material, beispielsweise einem Schaumstoffmaterial, gebildet sein kann, während das oben auf dem Rahmen angeordnete und von vorn sichtbare Zierelement aus rein gestalterischen Überlegungen heraus konstruiert sein kann, insbesondere aus gestalterisch besonders wirksamen Materialien gebildet sein kann. Anzumerken ist jedoch, dass das Zierelement prinzipiell auch selbst Teil des Anlagemittels sein kann oder ein Anlagemittel bilden kann, wobei dann gewisse Beschränkungen in der Designfreiheit des Zierelements in Kauf genommen werden müssen, andererseits jedoch durch die Doppelfunktion des Zierelements auf die zusätzliche Bereitstellung eines Anlagemittels verzichtet werden kann.

Als konstruktiv einfache und dabei sichere und optisch unauffällige Möglichkeit, das Zierelement an dem Rahmen zu halten, kann der Rahmen in dem mindestens einen Halteabschnitt eine zur Außengeometrie des an dem Halteabschnitt anliegenden Zierelementabschnitts komplementäre Formgebung oder ein das Zierelement formschlüssig haltendes Halteelement aufweisen. So kann vorteilhaft der Rahmen zumindest abschnittsweise an seiner Oberseite eine langgestreckte rinnenartige Vertiefung mit im Wesentlichen kreisabschnittsförmigem Querschnitt aufweisen, die sich in Verlaufsrichtung des oberen Rahmenabschnitts erstreckt, um in dieser Vertiefung ein längliches Zierelement mit im Wesentlichen rundem Querschnitt aufzunehmen. Auch andere Konstruktionen des mindestens einen Halteabschnitts sind denkbar, wobei die Halteabschnitte vorzugsweise so ausgebildet sind, dass sie den entsprechenden Abschnitt des Zierelements formschlüssig in Eingriff nehmen.

Alternativ oder zusätzlich zu der beschriebenen formschlüssigen Halterung des Zierelements am Rahmen kann zwischen dem Zierelement und dem Rahmen auch ein kraftschlüssiger Eingriff, d.h. ein Eingriff großenteils unter Reibwirkung, vorliegen. Insbesondere wird an eine Ausführungsform der Erfindung gedacht, in welcher das Zierelement unter Vorspannung an dem mindestens einen Halteabschnitt anliegt. Durch einen solchen kraftschlüssigen Halt wird einerseits eine einfache und dennoch sichere Verbindung zwischen Zierelement und Rahmen geschaffen, und andererseits auch die Montage, ggf. auch die Demontage des Zierelements erleichtert.

Eine Anlage des Zierelements unter Vorspannung kann auf einfache Weise bereitgestellt werden, wenn das Zierelement als geschlossener Ring ausgebildet ist. Das Zierelement kann dann den Rahmen entlang seiner Umlaufrichtung um den Durchsichtbereich vollständig umlaufen, wobei zur Herstellung der Vorspannung die umlaufende Länge des ringförmigen Zierelements geringfügig kleiner vorgesehen ist als die Länge der umlaufenden Anlagelinie des Rahmens.

Alternativ kann das Zierelement im Wesentlichen C-förmige Gestalt aufweisen, wodurch es möglich ist, etwa den Nasenbereich an der Rahmenunterseite der Sportbrille auszusparen, so dass das Zierelement den Rahmen oben, seitlich und ggf. unten seitlich umläuft. Mit einer solchen C-förmigen Gestalt kann ferner ein besonderer ästhetischer Effekt der Sportbrille erzielt werden. Diese Wirkung lässt sich darüber hinaus verstärken, wenn der obere Zierelementabschnitt entlang der Längserstreckungsrichtung des oberen Zierelementabschnitts zur Mitte hin, d.h. bei Annäherung an eine vertikale Mittellinie des Durchsichtbereichs, breiter wird. Eine solche Verbreiterung des mittleren Bereichs des oberen Zierelementabschnitts kann jedoch auch unabhängig von der C-förmigen Gestalt für andere Formgebungen des Zierelements den erwähnten ästhetischen Effekt entfalten.

Das Zierelement wird an dem mindestens einen Halteabschnitt an der Oberseite des Rahmens zumindest hinsichtlich seiner Lage abgestützt bzw. fixiert. Um den sicheren Halt des Zierelements am Rahmen der Sportbrille weiter zu verbessern, kann ein solcher Halteabschnitt jedoch auch als Befestigungsabschnitt ausgebildet sein, an welchem das Zierelement stabil befestigt ist, oder mindestens ein solcher Befestigungsabschnitt kann zusätzlich zu dem mindestens einen Halteabschnitt am Rahmen ausgebildet sein. In dem mindestens einen Befestigungsabschnitt kann das Zierelement unlösbar an dem Rahmen befestigt sein, beispielsweise über eine Klebe-oder Schweißverbindung oder über unlösbare Verbindungselemente, wie Niete, Klammern, Nähte oder dergleichen. Das Zierelement kann durch eine solche unlösbare Verbindung mit einfachen Mitteln besonders sicher an dem Rahmen gehalten werden. Alternativ kann jedoch auch eine lösbare Verbindung zwischen Zierelement und Rahmen vorgesehen sein, um die Auswechslung des Zierelements bei Beschädigung zu ermöglichen oder um für einen Rahmen verschiedene Zierelemente mit unterschiedlichen Gestaltungen verwenden zu können. Als lösbare Verbindungen kommen vorteilhaft Klemmverbindungen, aber auch Klettverbindungen, Schraubverbindungen oder Verbindungen durch elastischen Halt des Zierelements unter Vorspannung an dem Rahmen in Betracht.

In einer bevorzugten Ausführungsform ist vorgesehen, dass, entlang der Erstreckung des Zierelements betrachtet, das Zierelement in mindestens zwei im Abstand voneinander angeordneten Befestigungsabschnitten an dem Rahmen befestigt ist und mindestens ein Halteabschnitt zwischen den Befestigungsabschnitten angeordnet ist. Durch die Anordnung eines Halteabschnitts - in welchem das Zierelement zwar in gewisser Weise hinsichtlich seiner Position fixiert werden soll, jedoch nicht notwendigerweise verliersicher befestigt werden muss - zwischen zwei Befestigungsabschnitten ist es in dieser Ausführungsform möglich, die Rahmenstruktur in einem optisch besonders prominenten Bereich der Sportbrille, beispielsweise am oberen Rahmenabschnitt in der Brillenmitte, als konstruktiv einfachen und daher optisch unauffälligen Halteabschnitt auszubilden und gleichzeitig den sicheren Halt des Zierelements durch die an anderer Stelle des Rahmens bereitgestellten Befestigungsabschnitte zu gewährleisten.

In einer vorteilhaften Weiterbildung der zuletzt genannten Ausführungsform ist vorgesehen, dass die Sportbrille einen linken und einen rechten Kopfhalterungsabschnitt aufweist, an welchem jeweils ein Kopfhalterungsmittel ausgebildet, angebracht oder anschließbar ist, und dass je ein Befestigungsabschnitt am linken und am rechten Kopfhalterungsabschnitt vorgesehen ist. In dieser Weiterbildung können Bereiche am linken und rechten seitlichen Rand der Brille, in welchen auch die Kopfhalterungsabschnitte ausgebildet sind, relativ unauffällig zur Befestigung des Zierelements genutzt werden.

Ein Befestigungsabschnitt für das Zierelement kann auf besonders einfache und kostengünstige Weise mit einem Klemmabschnitt ausgeführt sein, in welchem das Zierelement zwischen elastischen Klemmmitteln eingeklemmt ist. Dabei kommt insbesondere ein Einklemmen des Zierelements zwischen zwei Klemmmitteln in Frage, welche materialeinheitlich mit dem Rahmen ausgebildet sind. Denkbar ist insbesondere, die Klemmmittel direkt beim Spritzgießen des Rahmens aus einem Kunststoffmaterial, beispielsweise TPU, zu formen, so dass für die Ausbildung der Befestigungsabschnitte kein separater Verfahrensschritt oder zusätzlicher Materialaufwand notwendig ist.

Durch die erfindungsgemäße Bereitstellung des Zierelements als separates Gestaltungsmerkmal der Brille sind der Designfreiheit des Zierelements nur wenig Grenzen gesetzt. Das Zierelement kann besonders vorteilhaft aus einem weicheren Material gebildet sein als der Rahmen, wodurch sich einerseits eine angenehme und hochwertig anmutende Handhabung der Sportbrille ergibt und andererseits ein gewisser Schutz der Sportbrille bei mechanischen Stößen, z.B. beim Herunterfallen, bereitgestellt wird.

In einer besonders bevorzugten Ausführungsform der Erfindung ist das Zierelement elastisch verformbar und im Wesentlichen unzerbrechlich. Es kann dann besonders vorteilhaft mit einem Rahmen, einem Durchsichtbereich sowie Kopfhalterungsmitteln kombiniert werden, welche allesamt aus elastisch verformbaren und ebenfalls im Wesentlichen unzerbrechlichen Materialien gebildet sind. Ein im Wesentlichen unzerbrechliches Zierelement und insbesondere seine Integration in einer insgesamt im Wesentlichen unzerbrechlichen Sportbrille verhindert eine Beschädigung der Brille auch bei starker oder langanhaltender mechanischer Beanspruchung, beispielsweise während des Transports oder der Aufbewahrung, während des Auf- und Absetzens oder während eines Sturzes. Die Merkmale dieser Ausführungsform ermöglichen somit einen unkomplizierten Umgang sowie eine lange Lebensdauer der Sportbrille.

Eine solche insgesamt im Wesentlichen unzerbrechliche Brille kann sich hinsichtlich Konstruktion und Materialien an der in der DE 10 2004 002 700 A1 beschriebenen elastischen Brille orientieren.

Um gewünschte optische oder/und haptische Eindrücke der Sportbrille zu erzielen, kann das Zierelement oder eine einen Kern umhüllende Außenschicht des Zierelements aus mindestens einem der folgenden Bestandteile gebildet sein: Textilmaterial, Leder, Kunstleder, Kunststoff, Gummi, Folie oder eine Kombination dieser. Insbesondere wird die Sportbrille visuell und haptisch besonders hochwertig anmuten, wenn die Sichtfläche des Zierelements mit mindestens zwei unterschiedlichen Materialien gestaltet ist oder/und Verzierungselemente trägt. Als Verzierungselemente kommen dabei verschiedene Schmucksteine, Applikationen aus Leder, Metall, Holz oder andere Verzierungen in Frage.

Alternativ oder zusätzlich zur Nutzung des Zierelements als Gestaltungsmerkmal der Sportbrille kann das Zierelement auch ein elektronisches Funktionsmodul aufweisen. Auf diese Weise wird eine einfache und sichere Befestigung eines Funktionsmoduls an der Sportbrille ermöglicht, so dass das Funktionsmodul verliersicher und optisch ansprechend bzw. unauffällig an der Sportbrille mitgeführt werden kann. Insbesondere kann in einer solchen Ausführungsform das Zierelement die vorteilhafte Doppelfunktion als Verzierung der Sportbrille einerseits und als Aufnahme des elektronischen Funktionsmoduls andererseits aufweisen.

Als elektronisches Funktionsmodul kommt hierbei ein elektronisches Medienwiedergabegerät (z. B. MP3-Player, Radio etc.), ein Telekommunikationsgerät, eine Anzeige- oder Beleuchtungseinrichtung aber auch passive oder aktive Sensoren, wie Transponder oder Reflektoren für Lawinenverschütteten-Suchsysteme (RECCO-Reflektoren), in Betracht. Besonders unauffällig und vor Stoß und anderen äußeren Einflüssen geschützt kann ein solches Funktionsmodul im Inneren des Zierelements untergebracht sein, wobei an geeigneter Stelle des Zierelements ggf. verschließbare Zugangsöffnungen vorgesehen sind, um einen Zugang zu dem Modul zu erlauben, oder Anschlüsse, beispielsweise für Kopfhörer, angeordnet sind. Besondere Flexibilität bietet eine erfindungsgemäße Sportbrille, bei welcher ein ein elektronisches Funktionsmodul aufweisendes Zierelement auswechselbar an dem Rahmen gehalten ist.

Der Durchsichtbereich der erfindungsgemäßen Sportbrille, welcher aus einem einzigen Sichtfenster für beide Augen oder aus zwei separaten Sichtfenstern aufgebaut sein kann, kann mit dem Rahmen prinzipiell fest verbunden sein. Um jedoch einen Austausch des Durchsichtbereichs zu ermöglichen und beispielsweise wahlweise verschiedene Durchsichtbereiche mit unterschiedlichen Durchsichteigenschaften (Tönungen, Farben, Beschichtungen etc.) in den Rahmen einsetzen zu können, ist jedoch in einer vorteilhaften Ausführungsform der erfindungsgemäßen Sportbrille der Durchsichtbereich lösbar mit dem Rahmen verbunden. Um eine solche lösbare Verbindung möglichst einfach und unauffällig zu gestalten, wird vorgeschlagen, dass die Sportbrille einen linken und einen rechten Kopfhalterungsabschnitt aufweist, an welchen ein Kopfhalterungsmittel ausgebildet, angebracht oder anschließbar ist, und dass am linken und am rechten Kopfhalterungsabschnitt jeweils lösbare Kopplungsmittel vorgesehen sind, mittels welchen der Durchsichtbereich und der Rahmen miteinander verbunden sind.

Eine besonders sichere und dennoch einfache Möglichkeit, den Durchsichtbereich und den Rahmen lösbar miteinander zu verbinden, wird darin gesehen, dass der Durchsichtbereich und der Rahmen jeweils mindestens ein Kopplungsloch aufweisen und dass in einem montierten Zustand der Sportbrille ein Kopplungsloch des Durchsichtbereichs und ein Kopplungsloch des Rahmens miteinander fluchten und ein Kopplungselement durch die Kopplungslöcher geführt ist.

Das genannte Kopplungselement kann dabei Teil einer Halterung für das Kopfhalterungsmittel sein, so dass durch die Doppelfunktion des Kopplungselements die Anzahl an Bauteilen bzw. Strukturelementen der Sportbrille reduziert werden kann. Insbesondere wird hier daran gedacht, dass die Halterung ein im Wesentlichen C-förmiger oder im Wesentlichen U-förmiger Haltebügel ist, dessen einer Schenkel durch die fluchtenden Kopplungslöcher geführt ist und an dessen anderem Schenkel ein Kopfhalterungsband angebracht ist. Eine solche Halterung ist besonders einfach aufgebaut und lässt sich mit einfachen Handgriffen bedienen.

Die Vorteile der vorliegenden Erfindung kommen besonders zum Tragen, wenn die Sportbrille eine Skibrille oder Snowboardbrille ist.

Die Erfindung wird nachfolgend anhand einer bevorzugten Ausführungsform unter Bezugnahme auf die beigefügten Zeichnungen näher erläutert.
- Fig. 1: zeigt eine perspektivische Ansicht eines Rahmens sowie eines zugehörigen Durchsichtbereichs für eine Sportbrille gemäß einer Ausführungsform der Erfindung bei Betrachtung von schräg vorn;
- Fig. 2: zeigt eine perspektivische Ansicht der in Fig. 1 abgebildeten Teile der Sportbrille von schräg hinten;
- Fig. 3: zeigt einen Haltebügel der Skibrille gemäß Fig. 1;
- Fig. 4: zeigt eine Ansicht der in Fig. 1 gezeigten Ausführungsform der Skibrille von vorn;
- Fig. 5: zeigt ein Zierelement für die Montage an der Skibrille gemäß Fig. 1, und
- Fig. 6: zeigt die Skibrille gemäß der Ausführungsform der Erfindung mit montiertem Zierelement.

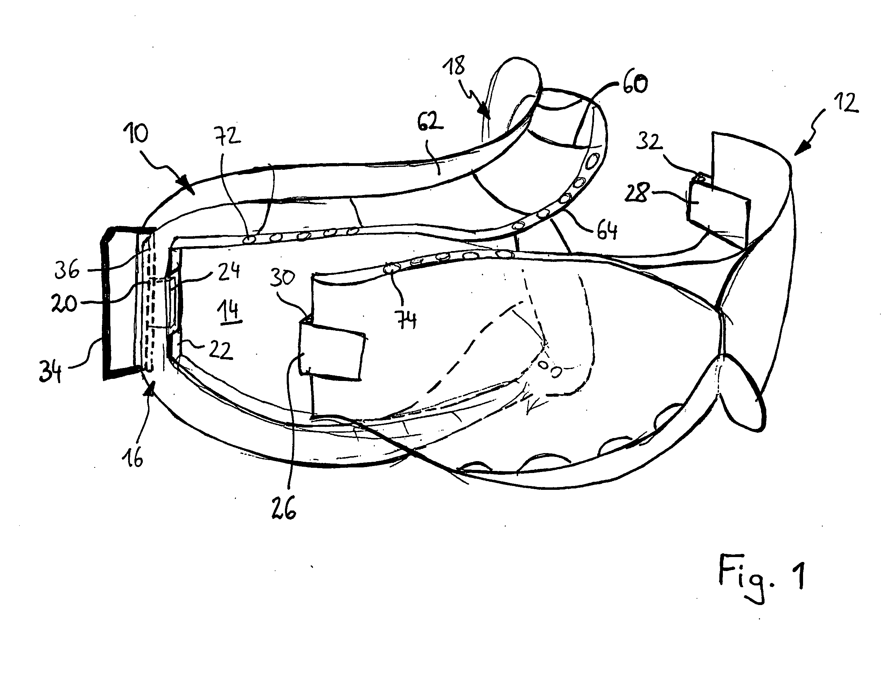

In Fig. 1 ist von einer Skibrille gemäß der Ausführungsform der Erfindung ein allgemein mit 10 bezeichneter Rahmen sowie ein allgemein mit 12 bezeichnetes Sichtfenster gezeigt. Der Rahmen 10 ist ein Spritzgussteil aus geeignetem Kunststoffmaterial, beispielsweise TPU oder einem anderen für die Herstellung von Sportbrillen an sich bekannten Material. Er ist als durchgehender Ring geformt und umläuft eine einzige Durchgangsöffnung 14 zur Aufnahme des Sichtfensters 12. Alternativ kann der Rahmen 10 zwei nebeneinander angeordnete Durchgangsöffnungen aufweisen, d.h. eine für jedes Auge, so dass der Rahmen 10 dann in Doppelringform gebildet ist.

Das Sichtfenster 12 ist aus einem transparenten Kunststoff (z.B. Polyurethan) gebildet und weist in an sich bekannter Weise eine gewünschte Tönung, Farbgebung oder Beschichtung auf.

Die Formgebung des Sichtfensters 12 sowie die des Rahmens 10 sind so aufeinander abgestimmt, dass das Sichtfenster 12 in die Durchgangsöffnung 14 des Rahmens 10 passt, so dass der Rahmen 10 am Rand des Sichtfensters 12 umlaufend anliegt.

Rahmen 10 und Sichtfenster 12 sind über ein Steckkopplungssystem lösbar miteinander verbunden. Hierzu weist der Rahmen 10 in einem rechten Begrenzungsabschnitt 16 sowie in einem linken Begrenzungsabschnitt 18, welche die Durchgangsöffnung 14 aus Sicht des Brillenträgers seitlich rechts und links begrenzen, jeweils einen Durchgang 20 auf, welcher den rechten bzw. linken Begrenzungsabschnitt der Länge nach in vertikaler Richtung durchdringt. Ferner mündet in die der Durchgangsöffnung 14 zugewandte Fläche 22 des rechten bzw. linken Begrenzungsabschnitts 16, 18 jeweils ein Schacht 24, welcher ebenfalls im Inneren des jeweiligen Begrenzungsabschnitts 16, 18 verläuft und mit dem Durchgang 20 verbunden ist.

Das Steckkopplungssystem umfasst ferner einen rechten und einen linken Vorsprung 26 bzw. 28, welche an den den Begrenzungsabschnitten 16, 18 zugeordneten seitlichen Endabschnitten des Sichtfensters 12 befestigt oder ausgebildet sind. Die Vorsprünge 26, 28 werden jeweils von einem Durchgang 30 bzw. 32 in vertikaler Richtung durchsetzt, deren Durchmesser gleich dem Durchmesser der Durchgänge 20 der Begrenzungsabschnitte 16, 18 des Rahmens 10 entspricht.

Die äußeren Abmessungen der Vorsprünge 26, 28 des Sichtfensters 12 sind den Innenabmessungen der Schächte 24 im Rahmen 10 angepasst, so dass sich die Vorsprünge 26, 28 in die Schächte 24 einführen lassen, wenn das Sichtfenster 12 auf den Rahmen 10 aufgesteckt wird. In diesem aufgesteckten Zustand sind die Durchgänge 30, 32 der Vorsprünge 26, 28 gegenüber den jeweiligen Durchgängen 20 im Rahmen 10 so positioniert, dass sie miteinander fluchten. Ein im Wesentlichen C-förmiger oder unterbrochen-ringförmiger Haltebügel 34 lässt sich dann mit einem ersten geraden Schenkel 36 durch die miteinander fluchtenden Durchgänge 20, 30 bzw. 32 des Rahmens 10 und des Sichtfensters 12 hindurchstecken, um so die Kopplung zwischen Rahmen 10 und Sichtfenster 12 zu arretieren. Zum Lösen der Kopplung kann der Haltebügel 34 vertikal wieder aus den Durchgängen 20, 30 bzw. 32 herausgezogen werden, woraufhin sich das Sichtfenster 12 nach vorn abnehmen lässt.

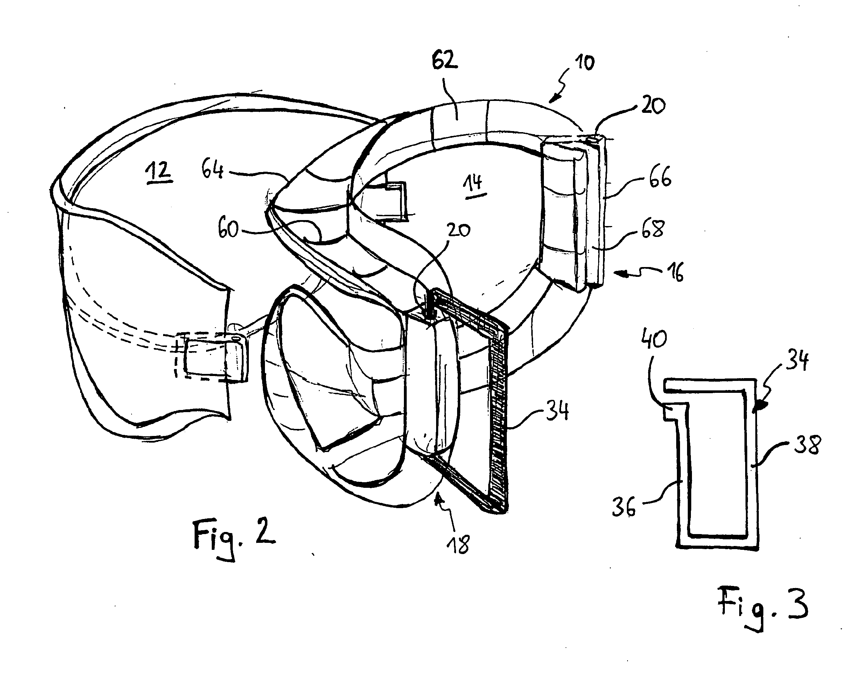

Der Haltebügel 34 ist deutlicher, jedoch ebenfalls nur in Prinzipdarstellung noch einmal in Fig. 3 gezeigt. Er umfasst zusätzlich zu dem ersten geraden Schenkel 36 einen gegenüberliegenden zweiten geraden Schenkel 38, an welchem ein Kopfhalteband der Skibrille anzubringen ist. Nur schematisch angedeutet ist in Fig. 3 ferner eine Clipverbindung 40, mit welcher der Haltebügel 34 nach der Montage des Kopfhaltebands sowie des Sichtfensters 12 zu einer geschlossenen Ringform zusammenklipsbar ist oder alternativ am Rahmen 10 anklipsbar ist. Sichtfenster 12, Rahmen 10 sowie das Kopfhalteband sind dann auf beiden Seiten über einen solchen Haltebügel 34 sicher miteinander verbunden.

Das Steckkopplungssystem zur Verbindung des Sichtfensters 12 mit dem Rahmen 10 umfasst vorzugsweise außerdem eine Anzahl von Schnappverbindungsmitteln, welche in den Figuren nicht dargestellt sind, um das Sichtfenster auch außerhalb der rechten und linken Begrenzungsabschnitte 16, 18 zu fixieren. Die Schnappverbindungsmittel sind aus Vorsprüngen im Randbereich des Sichtfensters 12 gebildet, die in entsprechende Ausnehmungen am Rahmen 10 eingeschnappt werden können. Dabei kommen an sich bekannte Schnappmechanismen mit Hinterschneidungen oder/und elastischen Elementen zum Einsatz, welche mit einer bestimmten, zwischen Rahmen 10 und Sichtfenster 12 ausgeübten Kraft ein- bzw. ausrasten, sofern der Haltebügel 34 herausgenommen ist.

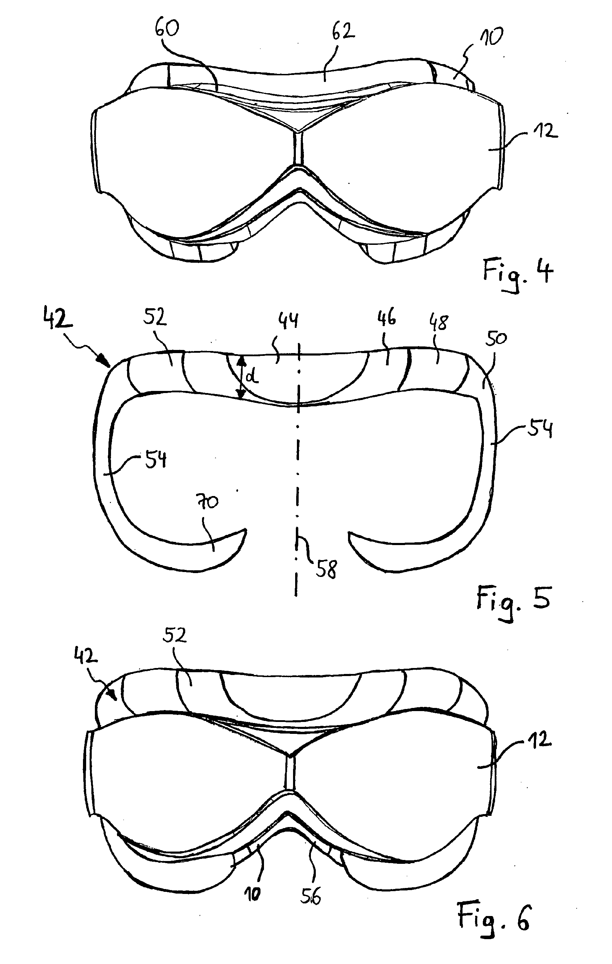

Unter Bezugnahme auf die Fig. 1, 2 sowie 4-6 werden nachfolgend ein wulstartiges Zierelement 42 sowie der Aufnahme und Befestigung des Zierelements 42 dienende Merkmale des Rahmens 10 näher beschrieben. Das in den Fig. 5 und 6 gezeigte Zierelement 42 ist ein flexibles, schlauchartiges Element mit annähernd rundem Querschnitt senkrecht zu seiner Erstreckungsrichtung. Es ist aus einem äußeren Mantel und einem von dem Mantel umhüllten Kern aus einem Polsterungsmaterial aufgebaut. In der vorliegenden Ausführungsform ist der Mantel des Zierelements 42 aus einer ersten Materialbahn 44 aus Leder sowie aus einer zweiten, einer dritten und einer vierten Materialbahn 46, 48 bzw. 50 aus unterschiedlichen Textilmaterialien zusammengesetzt, wobei die Materialbahnen 44-50 zu dem geschlossenen Mantel des Zierelements 42 zusammengenäht sind. Es ist jedoch auch eine beliebige andere Gestaltung des Zierelements 42 einschließlich einer zusätzlichen Verzierung mit Applikationen verschiedener Arten denkbar.

Das Zierelement 42 ist an dem Rahmen 10 der Sportbrille so angebracht, dass es den Rahmen 10 im Wesentlichen C-förmig umläuft. Dabei liegt ein oberer Zierelementabschnitt 52 des Zierelements 42, welcher in Erstreckungsrichtung des Zierelements 42 ein mittlerer Abschnitt ist, auf einer Oberseite des Rahmens 10 auf, während seitliche Abschnitte 54 des Zierelements 42, welche in Erstreckungsrichtung des Zierelements 42 Endabschnitte des Zierelements 42 sind, den Rahmen 10 hinter seinem rechten bzw. linken Begrenzungsabschnitt 16 bzw. 18 und weiter bis zur rechten bzw. linken Rahmenunterseite umlaufen. An einem mittleren unteren Abschnitt 56 des Rahmens 10 ist der Umlauf des Zierelements 42 um den Rahmen 10 unterbrochen.

Das Zierelement 42 ist entlang seiner Erstreckungsrichtung nicht durchweg mit konstantem Querschnittsdurchmesser d ausgebildet. Der Durchmesser d des Zierelements 42 nimmt im oberen Abschnitt 52 des Zierelements 42 mit abnehmender Entfernung von einer vertikalen Mittellinie 58 der Skibrille zu, ist also im mittleren Bereich am größten.

Um das Zierelement 42 in der erfindungsgemäßen Art an dem Rahmen 10 zu positionieren und zu befestigen, weist der Rahmen Halteabschnitte und Befestigungsabschnitte auf, welche im Folgenden unter Bezugnahme auf Fig. 1, 2 und 4 näher erläutert werden. Die Oberseite des Rahmens 10 ist durchweg konkav geformt. Das heißt, dass der Rahmen 10 an seinem sich zwischen den seitlichen Begrenzungsabschnitten 16, 18 erstreckenden oberen Rahmenabschnitt - in einem Querschnitt orthogonal zur Verlaufsrichtung des Rahmens betrachtet - eine im Wesentlichen U-förmige obere Begrenzungslinie zeigt. Diese Formgebung der Rahmenoberseite wird im Folgenden als Rinne bezeichnet und ist in Fig. 1, 2 und 4 mit dem Bezugszeichen 60 bezeichnet.

Die Rinne 60 geht an ihrem dem Kopf des Träger zugewandten Längsrand in eine Längswand 62 über, die von dem Rahmen 10 im Wesentlichen in vertikaler Richtung absteht. Der nach vorn weisende Rand 64 der Rinne 60 liegt dagegen frei. Die dem Kopf des Trägers zugewandte Rückseite der Längswand 62 ist mit einem in den Figuren nicht dargestellten Schaumstoffbelag versehen, welcher an der Stirn des Trägers anliegt.

Die Form der Oberseite des Rahmens 10, d.h. die Geometrie der Rinne 60 sowie der Längswand 62 sind auf die Außengeometrie des Zierelements 42 abgestimmt, so dass das Zierelement 42 in der durch Rinne 60 und Längswand 62 gebildeten Aufnahme passend anliegt und zumindest in seiner Position fixiert ist. Dementsprechend liegt das Zierelement 42 hauptsächlich mit seiner Unterseite und mit seiner Rückseite an dem Rahmen 10 an, während im Wesentlichen die gesamte Vorderseite sowie ein Großteil der Oberseite des Zierelements 42 bei Betrachtung von vorn sichtbar freiliegen und somit als Gestaltungsfläche verwendet werden können.

Die seitlichen Abschnitte 54 des Zierelements 42 verlaufen entlang der Rückseite der rechten und linken Begrenzungsabschnitte 16, 18 des Rahmens 10 und sind dort jeweils zwischen zwei Klemmbacken 66 eingeklemmt, wobei nur das rechte Klemmbackenpaar 66 in Fig. 2 illustriert ist. Die Klemmbacken 66 sind einstückig an den Begrenzungsabschnitten 16, 18 während des Spritzgießens des Rahmens 10 hergestellt. Sie bilden zwischen sich einen vertikal verlaufenden Klemmbereich 68, in welchen die seitlichen Zierelementabschnitte 54 eingelegt und durch die elastische Kraft der Klemmbacken 66 festgehalten werden können.

Die Klemmbacken 66 stellen den größten Teil der Befestigungskraft zur Befestigung des Zierelements 42 bereit. Zwischen dem linken Klemmbackenpaar 66 und dem rechten Klemmbackenpaar 66 ist das Zierelement 42 relativ straff gespannt und liegt so unter gewisser Vorspannung an der Rahmenoberseite an. An der Rahmenunterseite können die äußersten Enden 70 des Zierelements 42 ebenfalls eingeklemmt sein oder können mit anderen an sich bekannten Mitteln (z.B. Klebeverbindung, Klettverbindung etc.) an dem Rahmen 10 fixiert werden.

In Fig. 1 sind ferner rahmenseitige Belüftungslöcher 72 und fensterseitige Belüftungslöcher 74 angedeutet, welche in einem montierten Zustand der Skibrille übereinander liegen, um einen Luftaustausch des Brilleninneren mit der Außenumgebung zu ermöglichen. Ein solches Belüftungssystem vermeidet insbesondere ein Anlaufen des Sichtfensters.

Wie in Fig. 6 zu erkennen ist, ist das Zierelement 42 mit den vorstehend beschriebenen Halterungs- und Befestigungsmitteln so an dem Rahmen 10 abgestützt, dass der größte Teil der vorn liegenden Seite des oberen Zierelementabschnitts 52, d.h. der größte Teil des aus einer Ansicht gemäß Fig. 5 sichtbaren Oberflächenbereichs des oberen Zierelementabschnitts 52, bei Betrachtung der montierten Skibrille aus einer Richtung von vorn gemäß Fig. 6, sichtbar freiliegt. Insbesondere ist in der gezeigten Ausführungsform im Wesentlichen die gesamte vorn liegende Seite des oberen Abschnitts 52 des Zierelements 42, bei Betrachtung der montierten Skibrille von vorn, sichtbar freigelegt. Mit geringem Konstruktions- und Materialaufwand und somit bei geringen Kosten und bei niedrigem Gewicht kann so eine möglichst große und auffällige Gestaltungsfläche an der Skibrille bereitgestellt werden.

Ferner sind sowohl das Zierelement 42 als auch der Rahmen 10 und das Sichtfenster 12 aus im Wesentlichen unzerbrechlichen Materialien gebildet, sodass die gesamte Sportbrille zwar elastisch verformbar, jedoch im Wesentlichen unzerbrechlich ist. Bezüglich der Wahl der Materialien sowie weiterer hier nicht im Detail beschriebener Konstruktionsmerkmale orientiert sich die gezeigte Ausführungsform vorteilhaft auch an der in der DE 10 2004 002 700 A1 offenbarten, elastischen und im Wesentlichen unzerbrechlichen Brille.

## Patentansprüche

1. Sportbrille umfassend einen Durchsichtbereich (12) und einen den Durchsichtbereich (12) zumindest teilweise umlaufenden Rahmen (10),
**dadurch gekennzeichnet,**
**dass** an dem Rahmen (10) ein wulstartiges Zierelement (42) befestigt ist und
**dass** der Rahmen (10) zumindest an seiner Oberseite mindestens einen Halteabschnitt (60, 62) aufweist, welcher einen oberen Zierelementabschnitt (52) des Zierelements (42) derart abstützt, dass der größte Teil der vorn liegenden Seite des oberen Zierelementabschnitts (52) bei Betrachtung in einer zur Durchsichtrichtung der Sportbrille entgegengesetzten Richtung sichtbar freiliegt.

2. Sportbrille nach Anspruch 1, **dadurch gekennzeichnet, dass** an dem Rahmen (10) ferner ein von dem Zierelement (42) separates Anlagemittel (62) vorgesehen ist, welches dafür eingerichtet ist, in einem Benutzungszustand der Sportbrille zumindest an der Stirn einer die Sportbrille tragenden Person anzuliegen.

3. Sportbrille nach einem der vorhergehenden Ansprüche, **dadurch gekennzeichnet,**
**dass** der Rahmen (10) in dem mindestens einen Halteabschnitt (60, 62) eine zur Außengeometrie des an dem Halteabschnitt (60, 62) anliegenden Zierelementabschnitts (52) komplementäre Formgebung oder ein das Zierelement formschlüssig haltendes Halteelement aufweist.

4. Sportbrille nach einem der vorhergehenden Ansprüche, **dadurch gekennzeichnet,**
**dass** das Zierelement (42) unter Vorspannung an dem mindestens einen Halteabschnitt (60, 62) anliegt.

5. Sportbrille nach einem der vorhergehenden Ansprüche, **dadurch gekennzeichnet,**
**dass** das Zierelement (42) im Wesentlichen C-förmige Gestalt aufweist.

6. Sportbrille nach einem der vorhergehenden Ansprüche, **dadurch gekennzeichnet,**
**dass**, entlang der Erstreckung des Zierelements (42) betrachtet, das Zierelement (42) in mindestens zwei im Abstand voneinander angeordneten Befestigungsabschnitten (66) an dem Rahmen (10) befestigt ist und mindestens ein Halteabschnitt (60, 62) zwischen den Befestigungsabschnitten (66) angeordnet ist.

7. Sportbrille nach Anspruch 6, **dadurch gekennzeichnet,**
**dass** die Sportbrille einen linken und einen rechten Kopfhalterungsabschnitt (16, 18) aufweist, an welchem jeweils ein Kopfhalterungsmittel ausgebildet, angebracht oder anschließbar ist, und
**dass** je ein Befestigungsabschnitt (66) am linken und am rechten Kopfhalterungsabschnitt (16, 18) vorgesehen ist.

8. Sportbrille nach einem der vorhergehenden Ansprüche, **dadurch gekennzeichnet,**
**dass** mindestens ein Befestigungsabschnitt (66) des Zierelements (42) einen Klemmabschnitt (66) aufweist, in welchem das Zierelement (42) zwischen elastischen Klemmmitteln (66) eingeklemmt ist.

9. Sportbrille nach einem der vorhergehenden Ansprüche, **dadurch gekennzeichnet,**
**dass** das Zierelement (42) aus einem weicheren Material gebildet ist als der Rahmen (10).

10. Sportbrille nach einem der vorhergehenden Ansprüche, **dadurch**
**gekennzeichnet,**
**dass** das Zierelement (42) im Wesentlichen unzerbrechlich ist.

11. Sportbrille nach einem der vorhergehenden Ansprüche, **dadurch**
**gekennzeichnet,**
**dass** das Zierelement (42) oder eine einen Kern umhüllende Außenschicht des Zierelements (42) aus mindestens einem der folgenden Bestandteile gebildet ist: Textilmaterial, Leder, Kunstleder, Kunststoff, Gummi, Folie oder eine Kombination dieser.

12. Sportbrille nach einem der vorhergehenden Ansprüche, **dadurch**
**gekennzeichnet,**
**dass** die Sichtfläche des Zierelements (42) mit mindestens zwei unterschiedlichen Materialien gestaltet ist oder/und Verzierungselemente trägt.

13. Sportbrille nach einem der vorhergehenden Ansprüche, **dadurch**
**gekennzeichnet,**
**dass** der Durchsichtbereich (12) und der Rahmen (10) lösbar miteinander verbunden sind.

14. Sportbrille nach Anspruch 13, **dadurch gekennzeichnet,**
**dass** die Sportbrille einen linken und einen rechten Kopfhalterungsabschnitt (16, 18) aufweist, an welchen ein Kopfhalterungsmittel ausgebildet, angebracht oder anschließbar ist, und
**dass** am linken und am rechten Kopfhalterungsabschnitt (16, 18) jeweils lösbare Kopplungsmittel (24, 26, 28, 34) vorgesehen sind, mittels welchen der Durchsichtbereich (12) und der Rahmen (10) miteinander verbunden sind.

15. Sportbrille nach Anspruch 13 oder Anspruch 14, **dadurch gekennzeichnet,**
**dass** der Durchsichtbereich (12) und der Rahmen (10) jeweils mindestens ein Kopplungsloch (30, 32, 20) aufweisen und
**dass** in einem montierten Zustand der Sportbrille ein Kopplungsloch (30, 32) des Durchsichtbereichs (12) und ein Kopplungsloch (20) des Rahmens (10) miteinander fluchten und ein Kopplungselement (34) durch die Kopplungslöcher (30, 32, 20) geführt ist.

16. Sportbrille nach Anspruch 15, **dadurch gekennzeichnet,**
**dass** das Kopplungselement (34) ein im Wesentlichen C-förmiger oder im Wesentlichen U-förmiger Haltebügel (34) ist, dessen einer Schenkel (36) durch die fluchtenden Kopplungslöcher (20, 30, 32) geführt ist und an dessen anderem Schenkel (38) ein Kopfhalterungsband angebracht ist.

17. Sportbrille nach einem der vorhergehenden Ansprüche, **dadurch gekennzeichnet,**
**dass** die Sportbrille eine Skibrille oder Snowboardbrille ist.
